# EUROPÄISCHE PATENTSCHRIFT

(11) **EP 3 173 129 B1**
(45) Veröffentlichungstag und Bekanntmachung des Hinweises auf die Patenterteilung: **29.01.2020**
(21) Anmeldenummer: 16196695.7
(22) Anmeldetag: 01.11.2016
(51) Int. Cl.: A61Q 17/04, A61K 8/29, A61K 8/37

(54) **TITANDIOXID-HALTIGES SONNENSCHUTZMITTEL MIT ETHYLHEXYL SALICYLATE**
SUNSCREEN COMPRISING TITANIUM DIOXIDE AND ETHYLHEXYL SALICYLATE
COMPOSITION ANTI-SOLAIRE COMPRENANT DU DIOXIDE DE TITANIUM ET DE L'ETHYLHEXYL SALICYLATE

(30) Priorität: 25.11.2015 DE 102015223260
(43) Veröffentlichungstag der Anmeldung: 31.05.2017
(73) Patentinhaber: Beiersdorf AG, 20253 Hamburg (DE)
(72) Erfinder: Eitrich, Anja, 25337 Kölln-Reisiek (DE); Sprock, Sarah, 22297 Hamburg (DE); Schade, Juliane, 22529 Hamburg (DE); Göddertz, Dominik, 22763 Hamburg (DE); Eisert, Anja, 22085 Hamburg (DE); Reiter, Katharina, 21357 Barum (DE)

(56) Entgegenhaltungen:
- WO-A1-2015/044306
- WO-A2-2011/061133
- DE-A1-102012 205 526
- DE-A1-102014 207 602
- "Use of an agent for screening out light radiation with a wavelength ranging from 340 to 400nm", IP.COM JOURNAL, IP.COM INC., WEST HENRIETTA, NY, US, 5. Februar 2007 (2007-02-05), XP013117867, ISSN: 1533-0001
- MARIA EUGENIA CARLOTTI ET AL: "Role of particle coating in controlling skin damage photoinduced by titania nanoparticles", FREE RADICAL RESEARCH, Bd. 43, Nr. 3, 1. Januar 2009 (2009-01-01) , Seiten 312-322, XP055329199, GB ISSN: 1071-5762, DOI: 10.1080/10715760802716633
- E. GILBERT ET AL: "Commonly used UV filter toxicity on biological functions: review of last decade studies", INTERNATIONAL JOURNAL OF COSMETIC SCIENCE, 15. Januar 2013 (2013-01-15), Seiten n/a-n/a, XP055063284, ISSN: 0142-5463, DOI: 10.1111/ics.12030

## Beschreibung

Die vorliegende Erfindung betrifft eine kosmetische Zubereitung enthaltend Octylsalicylat (Ethylhexyl Salicylat) und Titandioxid in der Kristallstruktur Rutil mit einer Primärpartikelgröße von 2 -100 nm.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

So sind im Stande der Technik eine Vielzahl von Sonnenschutzmitteln bekannt, die als UV-Filter mikronisiertes Titandioxid enthalten. Dabei werden in der Regel Titandioxide eingesetzt, die ganz oder teilweise in der Kristallstruktur Anatas vorliegen, beispielsweise das Titandioxid mit dem Handelsnamen Tego Sun T 805 von Evonik, welches eine Mischung aus den Kristallstrukturen Anatas/Rutil von etwa 80:20 aufweist. Zubereitungen mit Titandioxid in Anatas-Struktur haben dabei den Vorteil, transparenter zu sein als vergleichbare Zubereitungen mit Titandioxid in Rutil-Struktur,

Nachteilig am Stande der Technik ist jedoch der Umstand, dass die Zubereitungen enthaltend Titandioxid in Anatas-Struktur relativ schlechte Filme auf der Haut bilden. Diese Filmbildung kann dabei experimentell im Labor mit Hilfe Fluoreszenzlöschmethode bestimmt werden.

Es war daher die Aufgabe der vorliegenden Erfindung, ein Sonnenschutzmittel auf der Basis von mikronisiertem Titandioxid zu entwickeln, bei dem die Filmbildung (d.h. die Ausbildung einer homogenen, möglichst lückenlosen Produktschicht auf der Haut) verbessert ist.

Nachteilig am Stande der Technik ist darüber hinaus der Umstand, dass viele Titandioxid enthaltende Sonnenschutzmittel bei der Lagerung über einen längeren Zeitraum zur Bildung von Gasbläschen neigen. Diese Gasbildung (Wasserstoff) tritt dabei auch bei beschichteten Titandioxiden immer wieder auf. Es war daher die Aufgabe der vorliegenden Erfindung, ein Sonnenschutzmittel auf der Basis von mikronisiertem Titandioxid zu entwickeln, bei dem die Gasbildung während der Lagerung wirkungsvoll unterdrückt ist.

Gelöst werden die Aufgaben durch eine kosmetische Zubereitung gemäß Anspruch 1.

Die Filmbildung lässt sich dabei mit Hilfe der Fluoreszenzlöschmethode wie folgt bestimmen; Eingesetzte Methode: Löschung von Papierfluoreszenz

Prinzip: Druckerpapieren werden optische Aufheller zugesetzt, die bei Bestrahlung mit UV-Licht blau fluoreszieren. Wenn ein Produktfilm mit UV-Schutz auf dem Papier liegt, wird die Fluoreszenz, je nach Stärke des UV-Schutzes, geschwächt oder ganz gelöscht. So kann der UV-Schutz auf einfache Weise beurteilt werden.

Durchführung: Für jede Emulsion wurde ein neuer und unbenutzter Latexfingerling auf den rechten Zeigefinger aufgezogen und 30 s mit der jeweiligen Emulsion eingerieben (Sättigung des Fingerlings mit der Emulsion). Anschließend wurde eine kleine Menge der jeweiligen Emulsion auf ein Stückchen Vitro Skin aufgetragen und ebenfalls 30 s lang mit dem behandschuhten Finger eingerieben (Einstellung Gleichgewicht Fingerling/Kunsthautoberfläche). Als nächster Schritt wurden 28,0 +/- 0,4 mg der entsprechenden Emulsion auf ein Vitro-Skin-Objektträger übertragen. Dabei wurde die Emulsion erst einmal punktförmig auf der künstlichen Haut verteilt Im Anschluss wurde die Emulsion erst mit kreisenden Bewegungen, dann jeweils in Quer- und Längsrichtung zum Objektträger eingerieben, wobei die "Einreibungsdauer" 30 s. betrug. Damit ergibt sich eine Auftragsmenge von 2 mg Emulsion / cm². Nach der Auftragung des letzten Objektträgers wurden alle Vitro-Skin-Objektträger 20 Minuten bei Raumtemperatur getrocknet. Im Anschluss an die Trocknungszeit wurden Aufnahmen mit einer Digitalkamera bei den verschiedenen Anregungsbereichen Tageslicht, 312 nm UVB, 366 nm UVA (2x6W UV-Lampe, Firma VILBER LOURMAT) angefertigt und anschließend per Bildauswertungssoftware (analySIS start, Firma Olympus) über die Blauwerte (RGB-Farbraum rot/grün/blau, jeweils 0 bis 255, horizontal über die Plattenbreite gemittelte Werte) ausgewertet.

Zwar kennt der Stand der Technik die DE 102012205526 A1, offenbarend Sonnenschutzmittel mit Titandioxiden unbekannter Kristallstruktur und anderer Beschichtung, die WO 2011/061133 A2, offenbarend Titandioxid-haltige Sonnenschutzmittel mit Propyl- und Butylparabenen und DE 102014 207602 A1, offenbarend Sonnenschutzmittel mit Titandioxid, welches mit Methicone beschichtet ist, doch konnten diese Schriften nicht den Weg zur vorliegenden Erfindung weisen.

Es ist erfindungsgemäß, wenn die erfindungsgemäße Zubereitung ausschließlich Titandioxid in der Kristallstruktur Rutil enthält. Dabei gilt erfindungsgemäß ein Titandioxid dann als ausschließlich in der Kristallstruktur vorliegend, wenn der Gehalt an anderen Kristallstrukturen in der Summe 1 Gewichts-%, bezogen auf die Gesamtmenge an Titandioxid, nicht überschreitet.

Es ist erfindungsgemäß bevorzugt, wenn die Primärpartikelgröße des erfindungsgemäßen Titandioxides im Bereich zwischen 5 und 50 nm liegt.

Erfindungsgemäße Ausführungsformen der vorliegenden Erfindung sind dabei dadurch gekennzeichnet, dass das Titandioxid mit Silica (Siliziumdioxid und/oder Kieselsäure) beschichtet ist.

Es ist dabei erfindungsgemäß, wenn das mit Silica beschichtete Titandioxid auf der äußeren Seite der Silica-Schicht (d.h. auf der dem Titandioxid-abgewandten Seite) eine Schicht aus Dimethicone aufweist.

Es ist erfindungsgemäß vorteilhaft, wenn die sekundäre Partikelgröße des Titandioxid in der Kristallstruktur Rutil mit einer Primärpartikelgröße von 2 -100 nm zwischen 0,05 und 50 µm beträgt. Dabei ist der Bereich von 0,1 bis 1 µm erfindungsgemäß bevorzugt.

Die primäre und sekundäre Partikelgröße entnimmt der Fachmann folgender Literaturstelle: SCCS/1516/13 Opinion on Titanium Dioxide (nano form) Colipa No S75 der Cosmetics Europe personal care association.

Es ist vorteilhaft im Sinne der vorliegenden Erfindung, wenn die Zubereitung Octylsalicylat (Ethylhexyl Salicylat) in einer Konzentration von 0,5 bis 5 Gewichts-%, bezogen auf das Gesamtgewicht der Zubereitung, enthält. Dabei wird ein Gehalt von 2 bis 4,75 Gewichts-%, bezogen auf das Gesamtgewicht der Zubereitung, erfindungsgemäß bevorzugt.

Erfindungsgemäß vorteilhafte Ausführungsformen der vorliegenden Erfindung sind dadurch gekennzeichnet, dass die Zubereitung das Titandioxid in der Kristallstruktur Rutil mit einer Primärpartikelgröße von 2 -100 nm in einer Konzentration von 0,5 bis 10 Gewichts-%, bezogen auf das Gesamtgewicht der Zubereitung, enthält. Dabei wird ein Gehalt von 1 bis 6 Gewichts-%, bezogen auf das Gesamtgewicht der Zubereitung, erfindungsgemäß bevorzugt.

Die erfindungsgemäße kosmetische Zubereitung kann in unterschiedlichen Formen vorliegen, beispielsweise in Form einer Emulsion oder Hydrodispersion. Dabei ist es erfindungsgemäß vorteilhaft, wenn die Zubereitung in Form einer Emulsion vorliegt. Erfindungsgemäß bevorzugt ist es dabei, wenn die Zubereitung in Form einer O/W-Emulsion (Öl-in-Wasser-Emulsion) vorliegt.

Liegt die erfindungsgemäße kosmetische Zubereitung in Form einer Emulsion (insbesondere O/W-Emulsion) vor, so ist es erfindungsgemäß vorteilhaft, wenn die Zubereitung einen oder mehrere Emulgatoren enthält, die gewählt werden aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Natriumstearoylglutamat, Polyglyceryl-10 stearat, Cetearylalkohol, Cetearylsulfosuccinat, Kaliumcetylphosphat, enthält. Erfindungsgemäß bevorzugt sind dabei die Emulgatoren Glycerylstearat (selbstemulgierend), Stearinsäure, Stearatsalze, Polyglyceryl-3-methylglycosedistearat, Natriumcetearylsulfat, Natriumstaroylglutamat, Polyglyceryl-10 stearat, Cetearylsulfosuccinat.

Erfindungsgemäß vorteilhaft beträgt der Gesamtgehalt dieser Emulgatoren von 0,1 bis 3 Gewichts-%, bezogen auf das Gesamtgewicht der Zubereitung, wobei dieses Gesamtgewicht dem Einzelgewicht eines Emulgators entspricht für den Fall, dass nur eine dieser Verbindungen in der Zubereitung eingesetzt wird.

Ferner zeichnen sich erfindungsgemäß vorteilhafte Ausführungsformen der vorliegenden Erfindung dadurch aus, dass die Zubereitung ein oder mehrere Alkohole gewählt aus der Gruppe der Verbindungen Ethanol, 1,2-Propylenglycol, 1,2-Butylenglycol, 1,3-Butylenglycol, 1,2-Pentandiol, 1,2-Hexandiol, 1,2-Octandiol, 1,2-Decandiol, 2-Methyl-1,3-propandiol Phenoxyethanol und/oder Ethylhexylglycerin enthält, wobei der Einsatz von Ethanol, 1,2-Pentandiol, 1,2-Hexandiol, 1,2-Octandiol, 1,2-Decandiol, 2-Methyl-1,3-propandiol Phenoxyethanol und/oder Ethylhexylglycerin erfindungsgemäß bevorzugt ist. Eine alternative bevorzugte Ausführungsform mit Alkoholen ist die Kombination aus 1,2-Propylenglycol, 1,2-Butylenglycol und/oder 1,3-Butylenglycol mit 1,2-Octandiol.

Erfindungsgemäß vorteilhaft beträgt der Gesamtgehalt dieser dieser Alkohole von 0,1 bis 5 Gewichts-%, bezogen auf das Gesamtgewicht der Zubereitung, wobei dieses Gesamtgewicht dem Einzelgewicht eines Alkohols entspricht für den Fall, dass nur eine dieser Verbindungen in der Zubereitung eingesetzt wird.

Erfindungsgemäß vorteilhafte Ausführungsformen der vorliegenden Erfindung sind auch dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; Terephthaliden-dicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-Bis-{[4-(2-ethyl-hexyloxy)-2-hydroxy]-phenyl}-6-(4-methoxyphenyl)-1,3,5-triazin; 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate; Zinkoxid.

Erfindungsgemäß bevorzugt enthält die Zubereitung 4-(tert.-Butyl)-4'-methoxydibenzoylmethan und/oder 4-(Dimethylamino)-benzoesäure(2-ethylhexyl)ester.

Die erfindungsgemäß vorteilhafte Gesamtkonzentration dieser beiden UV-Filter beträgt von 0,5 bis 5 Gewichts-%, bezogen auf das Gesamtgewicht der Zubereitung, wobei dieses Gesamtgewicht dem Einzelgewicht entspricht für den Fall, dass nur eine dieser Verbindungen in der Zubereitung eingesetzt wird.

Erfindungsgemäß bevorzugt ist es auch, wenn die Zubereitung 2,4,6-Tris-[anilino-(p-carbo-2'-ethyl-1'-hexyloxy)]-1,3,5-triazin (INCI: Ethylhexyl Triazone) und/oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) enthält.

In einem solchen Falle beträgt die erfindungsgemäß vorteilhafte Gesamtkonzentration dieser beiden UV-Filter beträgt von 0,5 bis 8 Gewichts-%, bezogen auf das Gesamtgewicht der Zubereitung, wobei dieses Gesamtgewicht dem Einzelgewicht entspricht für den Fall, dass nur eine dieser Verbindungen in der Zubereitung eingesetzt wird.

Erfindungsgemäß vorteilhafte Ausführungsformen sind dadurch gekennzeichnet, dass die Emulsion Pirocton-Olamin (1-Hydroxy-4-methyl-6-(2,4,4-trimethylpentyl)- 2(1*H*)-pyridon monoethanolaminsalz) enthält.

Enthält die Emulsion Pirocton-Olamin so ist es erfindungsgemäß von Vorteil, wenn der Gehalt an Pirocton-Olamin von 0,01 bis 1 Gew.-%, bezogen auf das Gesamtgewicht der Emulsion beträgt.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat und/oder C12-C15 Alkylbenzoat enthält.

Darüber hinaus kann die erfindungsgemäße Zubereitung die üblichen für derartige Zubereitungen bekannten lipophilen Inhaltsstoffe enthalten, beispielsweise Öle, Fette Wachse und dergleichen.

Darüber hinaus ist es erfindungsgemäß von Vorteil, wenn die Zubereitung eine oder mehrere Verbindungen gewählt aus der Gruppe der Verbindungen Gylcyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivat, Glycyrrhiza Inflata Root Extract, Polydocanol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze, Licochalcon A, enthält.

Die Wasserphase der erfindungsgemäßen Emulsion kann übliche kosmetische Hilfsstoffe enthalten, wie beispielsweise Glycerin, Elektrolyte, Selbstbräuner sowie ein oder mehrere Verdickungsmittel.

Erfindungsgemäß bevorzugt ist es dabei, wenn die kosmetische Zubereitung dadurch gekennzeichnet ist, dass sie, bezogen auf das Gesamtgewicht der Zubereitung 5 bis 15 Gew.-% Glycerin enthält.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Es ist ferner vorteilhaft im Sinne der vorliegenden Erfindung, wenn die erfindungsgemäße Zubereitung einen oder mehrere Parfümstoffe enthält. Diese können beispielsweise vorteilhaft gewählt werden aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Ethylenbrassylat, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im Wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 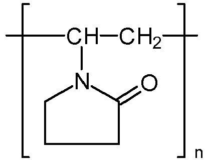 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79), Acrylates Copolymer (Epitex 66). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die erfindungsgemäße Zubereitung kann vorteilhaft als Creme, Lotion, Spray oder Stift-förmige Zubereitung dargereicht werden.

Erfindungsgemäß ist ihr Einsatz als Tagespflegeprodukt oder Sonnenschutzmittel.

Erfindungsgemäß ist nicht zuletzt die Verwendung des erfindungsgemäßen Titandioxides in der Kristallstruktur Rutil mit einer Primärpartikelgröße von 2 -100 nm zur Verbesserung der Filmbildungseigenschaften kosmetischer Zubereitungen enthaltend Ethylhexylsalicylat sowie die die Verwendung des erfindungsgemäßen Titandioxides in der Kristallstruktur Rutil mit einer Primärpartikelgröße von 2 -100 nm zur Reduzierung der Gasbildung in kosmetischen Zubereitungen enthaltend Ethylhexylsalicylat und Titandioxid.

### Vergleichsversuch

Mit Hilfe des folgenden Vergleichsversuches konnte der erfinderische Effekt beispielhaft belegt werden:

| | **PMCCBB10 138** | **PMCCBB10 140** |
|---|---|---|
| **INCI** | **m [%]** | **m [%]** |
| Ethylparaben | 0,20 | 0,20 |
| Methylparaben | 0,30 | 0,30 |
| Caprylic/Capric Triglyceride | 7,00 | 7,00 |
| Aqua | ad 100 | add 100 |
| Tocopheryl Acetate | 0,50 | 0,50 |
| Cetyl Alcohol | 0,50 | 0,50 |
| Trisodium EDTA | 0,20 | 0,20 |
| Phenoxyethanol | 0,50 | 0,50 |
| Hydrogenated Coco-Glycerides | 1,00 | 1,00 |
| Myristyl Myristate | 1,00 | 1,00 |
| Panthenol | 1,00 | 1,00 |
| VP/Hexadecene Copolymer | 0,50 | 0,50 |
| Glycerin | 8,50 | 8,50 |
| Sodium Hydroxide | 0,04 | 0,04 |
| Alcohol Denat. | 4,00 | 4,00 |
| Ethylhexyl Salicylate | 1,00 | 1,00 |
| Xanthan Gum | 0,30 | 0,30 |
| Acrylates/C10-30 Alkyl Acrylate Crosspolymer | 0,15 | 0,15 |
| Stearyl Alcohol | 0,50 | 0,50 |
| Glyceryl Stearate Citrate | 2,00 | 2,00 |
| Ethylhexylglycerin | 0,30 | 0,30 |
| Parfum | 0,40 | 0,40 |
| C12-15 Alkyl Benzoate | 7,00 | 7,00 |
| Titanium Dioxide (Anastase-Typ) + Trimethoxycaprylylsilane | | 3,00 |
| Titanium Dioxide (Rutil-Typ) + Silica + Dimethicone | 3,00 | |

### Methode: Löschung von Papierfluoreszenz, Doppelbestimmung

Die Methodenbeschreibung findet sich auf Seite 2-3
Die Auswertung erfolgt über die Anregung bei 312 nm (UVB), wobei gilt, dass die Fluoreszenzlöschung je größer ist, je geringer der verbleibende Blauwert ist.

### Proben: s. Tabelle oben, PMCCBB10#138 und #140

### Ergebnisse:

Werden die 312 nm-Aufnahmen (Unterschiede in diesen am deutlichsten erkennbar) per Bildauswertungssoftware über die Blauwerte* ausgewertet, erhält man folgende Werte:

| **Ansatz** | | **Blauwert** | **Blauwert [%]** *bezogen auf den Blindwert* | **Mittelwert Blauwert [%]** *bezogen auf den Blindwert* |
|---|---|---|---|---|
| Blindwert (ohne Emulsion) | 1 | 253,2 | 100,0 | 100,0 |
| | 2 | 241,4 | 100,0 | |
| #138 | 1 | 174,7 | 69,0 | 63,8 |
| | 2 | 141,2 | 58,5 | |
| #140 | 1 | 214,0 | 84,5 | 78,5 |
| | 2 | 174,9 | 72,5 | |

| | | | | |
|---|---|---|---|---|
| * RGB-Farbraum (rot/grün/blau, jeweils 0 bis 255), horizontal über die Plattenbreite gemittelte Werte | | | | |

Anhand dieser Werte lässt sich deutlich erkennen, dass die Fluoreszenzlöschung von Ansatz #138 (mit Titandioxid Rutil-Typ) stärker ausgeprägt/besser ist als bei Ansatz #140 (mit Titandioxid Anastase-Typ).

Das bedeutet, dass der Ansatz #138 (mit Titandioxid Rutil-Typ) eine deutlich bessere Filmbildung bewirkt, als der vergleichend getestete Ansatz #140 (mit Titandioxid Anastase-Typ).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **O/W Lotions** | **1** | **2** | **3** | **4** | **5** |
|---|---|---|---|---|---|
| **INCI** | **m [%]** | **m [%]** | **m [%]** | **m [%]** | **m [%]** |
| Tocopheryl Acetate | 0,06 | 0,10 | 0,10 | 0,15 | 0,50 |
| Panthenol | 1,00 | 1,40 | | 0,50 | 1,00 |
| Ethylhexylglycerin | 0,30 | 0,50 | 0,25 | | |
| 1,2-Hexanediol | | | | 0,40 | |
| Methylpropanediol | | | | | 0,20 |
| Caprylic/Capric Triglyceride | | 4,00 | | 2,00 | |
| Isopropyl Palmitate | | | | | 3,00 |
| Hydrogenated Coco-Glycerides | 1,00 | 2,00 | 9,00 | 1,00 | |
| Dimethicone | | | 1,00 | 0,50 | |
| Octyldodecanol | | | | 2,00 | |
| Ethylhexyl Cocoate | | | | 2,00 | |
| C18-38 Alkyl Hydroxystearoyl Stearate | | | 0,50 | | |
| Cetearyl Alcohol | | | | 0,50 | |
| Myristyl Myristate | 2,00 | 1,00 | 1,50 | | |
| C12-15 Alkyl Benzoate | 4,50 | 6,00 | | | |
| Glyceryl Stearate Citrate | 2,00 | 3,00 | | | |
| Natrium Cetearyl Sulfate | | | 0,15 | | |
| Polyglyceryl-3 Methylglucose Distearate | | | | | 0,15 |
| Glyceryl Stearate SE | | | 1,00 | | |
| Natrium Stearoyl Glutamate | | | | 0,50 | |
| Glyceryl Stearate | | | | 1,00 | |
| Silica Dimethyl Silylate | | 0,50 | 0,50 | 0,30 | 1,00 |
| Tapioca Starch | | | | 3,00 | 3,00 |
| VP/Hexadecene Copolymer | 0,50 | 1,00 | | | |
| Parfum | 0,40 | 0,40 | 0,30 | | 0,20 |
| Glycerin | 6,00 | 8,00 | 5,00 | 1,00 | 1,00 |
| Citric Acid | 0,07 | 0,09 | | | |
| Natrium Citrate | 0,16 | 0,17 | | | |
| Natrium Hydroxide | 0,05 | 0,08 | 0,30 | | 0,20 |
| Ethylparaben | 0,20 | 0,30 | 0,20 | | |
| Methylparaben | 0,20 | 0,30 | 0,30 | | |
| Phenoxyethanol | 0,30 | 0,50 | 0,40 | | 0,50 |
| Cetyl Alcohol | 0,50 | 1,00 | | | |
| Cetearyl Alcohol | | | 1,50 | | |
| Xanthan Gum | 0,30 | 0,50 | 0,40 | 0,30 | |
| Acrylates/C10-30 Alkyl Acrylate Crosspolymer | 0,10 | 0,15 | 0,05 | | 0,20 |
| Carbomer | | | | | 0,15 |
| Hydroxyethylcellulose | | | | | 0,10 |
| Stearyl Alcohol | 0,50 | 0,80 | | | |
| Aqua | ad 100 | ad 100 | ad 100 | ad 100 | ad 100 |
| Alcohol Denat. | 4,00 | 6,00 | 4,00 | 8,00 | 10,00 |
| Trinatrium EDTA | 1,00 | 0,50 | 1,00 | 1,00 | 1,00 |
| Butyl Methoxydibenzoylmethane | 4,50 | 1,50 | 4,75 | 4,00 | 4,00 |
| Ethylhexyl Salicylate | 4,50 | 3,00 | 4,75 | 4,50 | 4,00 |
| Phenylbenzimidazole Sulfonic Acid | | | 1,00 | | |
| Bis-Ethylhexyloxyphenol Methoxyphenyl Triazine | | | 3,50 | | |
| Octocrylene | 9,00 | 5,00 | 9,75 | 9,00 | 8,00 |
| Homosalate | 9,00 | 4,00 | 9,75 | | 8,00 |
| Polysilicone-15 | | | | | 1,00 |
| Titanium Dioxide (nano) + Silica + Dimethicone (Rutil-Typ) | 3,00 | 0,50 | 3,75 | 1,00 | 1,00 |

| **O/W Hydrodispersionen** | **1** | **2** | **3** | **4** | |
|---|---|---|---|---|---|
| **INCI** | **m [%]** | **m [%]** | **m [%]** | **m [%]** | |
| Tocopheryl Acetate | 0,10 | 0,10 | 0,50 | 0.50 | |
| Panthenol | 1,00 | 1,00 | 1,00 | 0,50 | |
| Ethylhexylglycerin | | | 0,25 | 0,30 | |
| Dibutyl Adipate | | | | 2,00 | |
| 2-Methyl-1,3-propandiol | | | | 0,02 | |
| C12-15 Alkyl Benzoate | 9,00 | | | | |
| C18-36 Acid Triglyceride | | 1,00 | 1,50 | | |
| Butylene Glycol Dicaprylate/Dicaprate | | 2,00 | 3,00 | | |
| Polyglyceryl-3 Methylglucose Distearate | 0,20 | | | 0,40 | |
| Sucrose Polystearate + Hydrogenated Polyisobutene | | | | 1,00 | |
| Sodium Stearoyl Glutamate | | | | | |
| Ceteareth-20 | | 1,50 | 1,00 | | |
| Silica Dimethyl Silylate | 0,50 | | | | |
| Microcrystalline Cellulose + Cellulose Gum | | | | 1,00 | |
| VP/Hexadecene Copolymer | 0,50 | 1,00 | 1,00 | 2,00 | |
| Parfum | 0,40 | 0,30 | | 0,20 | |
| Glycerin | 5,00 | 5,00 | 3,00 | 5,00 | |
| Butylene Glycol | | | 4,00 | | |
| Natrium Hydroxide | 0,10 | 0,50 | 0,40 | 0,2 | |
| Ethylparaben | 0,20 | 0,20 | | | |
| Methylparaben | 0,30 | 0,30 | | | |
| Phenoxyethanol | 0,50 | 0,50 | | 0,50 | |
| Xanthan Gum | 0,05 | | | 0,30 | |
| Acrylates/C10-30 Alkyl Acrylate Crosspolymer | 0,20 | | 0,30 | | |
| Aqua | ad 100 | ad 100 | ad 100 | ad 100 | |
| Alcohol Denat. | 5,00 | 4,00 | 6,00 | 4,00 | |
| Trinatrium EDTA | 0,40 | 0,80 | 0,80 | 0,80 | |
| Butyl Methoxydibenzoylmethane | 2,50 | 4,50 | 4,50 | 4,75 | |
| Phenylbenzimidazole Sulfonic Acid | | 1,50 | 1,00 | 1,00 | |
| Ethylhexyl Salicylate | 4,50 | 4,75 | 4,50 | 4,00 | |
| Octocrylene | 4,50 | 8,00 | 8,00 | 0,50 | |
| Ethylhexyl Triazone | | | | 1,00 | |
| Homosalate | | 9,00 | 9,00 | 7,00 | |
| Bis-Ethylhexyloxyphenol Methoxyphenyl Triazine | | 3,50 | 3,50 | 3,00 | |
| Titanium Dioxide (nano) + Silica + Dimethicone (Rutil-Typ) | 0,50 | 1,00 | 0,80 | 0,50 | |

## Patentansprüche

1. Kosmetische Zubereitung enthaltend
a) Octylsalicylat (Ethylhexyl Salicylat),
b) Titandioxid in der Kristallstruktur Rutil mit einer Primärpartikelgröße von 2 -100 nm, wobei die Zubereitung ausschließlich Titandioxid in der Kristallstruktur Rutil enthält und das Titandioxid mit Silica beschichtet ist, wobei das mit Silica beschichtete Titandioxid auf der äußeren Seite der Silica-Schicht eine Schicht aus Dimethicone aufweist, **dadurch gekennzeichnet, dass** die Zubereitung frei ist von Propylparaben, Butylparaben, Methylisothiazolinon, Chlormethylisothiazolinon, IPBC (IUPAC: 3-lod-2-propinylbutylcarbamat), DMDM-Hydantoin, Dimethylol Glycol, Dimethylol Urea, Sodium Hydroxmethyl Glycinate, BHT (IUPAC: 2,6-Di-*tert*-butyl-4-methylphenol), 3-(4-Methylbenzyliden)campher und 2-Hydroxy-4-methoxybenzophenon.

2. Kosmetische Zubereitung nach Anspruch 1, **dadurch gekennzeichnet, dass** die Zubereitung Octylsalicylat (Ethylhexyl Salicylat) in einer Konzentration von 0,5 bis 5 Gewichts-%, bezogen auf das Gesamtgewicht der Zubereitung, enthält.

3. Kosmetische Zubereitung nach einem der vorhergehenden Ansprüche, **dadurch gekennzeichnet, dass** die sekundäre Partikelgröße des Titandioxid in der Kristallstruktur Rutil mit einer Primärpartikelgröße von 2 -100 nm zwischen 0,05 und 50 µm beträgt.

4. Kosmetische Zubereitung nach einem der vorhergehenden Ansprüche, **dadurch gekennzeichnet, dass** die Zubereitung das Titandioxid in der Kristallstruktur Rutil mit einer Primärpartikelgröße von 2 -100 nm in einer Konzentration von 0,5 bis 10 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung in Form einer Emulsion vorliegt.

6. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Natriumstaroylglutamat, Polyglyceryl-10 stearat, Cetearylalkohol, Cetearylsulfosuccinat, Kaliumcetylphosphat, enthält

7. Kosmetische Zubereitung nach einem der vorhergehenden Ansprüche, **dadurch gekennzeichnet, dass** die Zubereitung ein oder mehrere Alkohole gewählt aus der Gruppe der Verbindungen Ethanol, 1,2-Pentandiol, 1,2-Hexandiol, 1,2-Octandiol, 1,2-Decandiol, 2-Methyl-1,3-propandiol, Phenoxyethanol und/oder Ethylhexylglycerin enthält.

8. Kosmetische Zubereitung nach einem der vorhergehenden Ansprüche, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; Terephthaliden-dicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-Bis-{[4-(2-ethyl-hexyloxy)-2-hydroxy]-phenyl}-6-(4-methoxyphenyl)-1,3,5-triazin; 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate; Zinkoxid.

9. Kosmetische Zubereitung nach einem der vorhergehenden Ansprüche, **dadurch gekennzeichnet, dass** die Zubereitung 2, 4, 6-Tris-[anilino-(p-carbo-2'-ethyl-1'-hexyloxy)]-1,3,5-triazin (INCI: Ethylhexyl Triazone) und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) enthält.

10. Kosmetische Zubereitung nach einem der vorhergehenden Ansprüche, **dadurch gekennzeichnet, dass** die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

11. Kosmetische Zubereitung nach einem der vorhergehenden Ansprüche, **dadurch gekennzeichnet, dass** die Zubereitung eine oder mehrere Verbindungen gewählt aus der Gruppe der Verbindungen Gylcyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivat, Glycyrrhiza Inflata Root Extract, Polydocanol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze, Licochalcon A, enthält.

## Claims

1. Cosmetic preparation comprising
a) octyl salicylate (ethylhexyl salicylate),
b) titanium dioxide in the rutile crystal structure with a primary particle size of 2-100 nm,
wherein the composition contains exclusively titanium dioxide in the rutile crystal structure and the titanium dioxide has been coated with silica, where the silica-coated titanium dioxide has a layer of dimethicone on the outside of the silicon layer, **characterized in that** the formulation is free of propylparaben, butylparaben, methylisothiazolinone, chloromethylisothiazolinone, IPBC (IUPAC: 3-iodo-2-propynyl butylcarbamate), DMDM hydantoin, dimethylol glycol, dimethylol urea, sodium hydroxymethyl-glycinate, BHT (IUPAC: 2,6-di-tert-butyl-4-methylphenol), 3-(4-methylbenzylidene)camphor and 2-hydroxy-4-methoxybenzophenone.

2. Cosmetic preparation according to Claim 1, **characterized in that** the formulation contains octyl salicylate (ethylhexyl salicylate) in a concentration of 0.5% to 5% by weight, based on the total weight of the preparation.

3. Cosmetic preparation according to either of the preceding claims, **characterized in that** the secondary particle size of the titanium dioxide in the rutile crystal structure with a primary particle size of 2-100 nm is between 0.05 and 50 µm.

4. Cosmetic preparation according to any of the preceding claims, **characterized in that** the formulation contains the titanium dioxide in the rutile crystal structure with a primary particle size of 2-100 nm in a concentration of 0.5% to 10% by weight, based on the total weight of the preparation.

5. Cosmetic preparation according to any of the preceding claims, **characterized in that** the preparation is in the form of an emulsion.

6. Cosmetic preparation according to any of the preceding claims, **characterized in that** the preparation comprises one or more emulsifiers selected from the group of compounds glyceryl stearate citrate, glyceryl stearate (self-emulsifying), stearic acid, stearate salts, polyglyceryl-3-methylglycose distearate, sodium cetearyl sulfate, sodium stearoyl glutamate, polyglyceryl-10 stearate, cetearyl alcohol, cetearyl sulfosuccinate, potassium cetyl phosphate.

7. Cosmetic preparation according to any of the preceding claims, **characterized in that** the preparation comprises one or more alcohols selected from the group of compounds ethanol, pentane-1,2-diol, hexane-1,2-diol, octane-1,2-diol, decane-1,2-diol, 2-methylpropane-1,3-diol, phenoxyethanol and/or ethylhexylglycerol.

8. Cosmetic preparation according to any of the preceding claims, **characterized in that** the preparation comprises one or more UV filters selected from the group of compounds 2-phenylbenzimidazole-5-sulfonic acid and/or salts thereof; phenylene-1,4-bis(2-benzimidazyl)-3,3';5,5'-tetrasulfonic acid salts; 1,4-di(2-oxo-10-sulfo-3-bornylidenemethyl)-benzene and salts thereof; 4-(2-oxo-3-bornylidenemethyl)benzenesulfonic acid salts; 2-methyl-5-(2-oxo-3-bornylidenemethyl)sulfonic salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]-propyl]phenol; 3-(4-methylbenzylidene)camphor; 3-benzylidenecamphor; 4-(tert-butyl)-4'-methoxydi-benzoylmethane; terephthalidenedicamphorsulfonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl 2-hydroxybenzoate; dimethicodiethyl benzalmalonate; 3-(4-(2,2-bisethoxycarbonylvinyl)-phenoxy)propenyl)methoxysiloxane/dimethylsiloxane copolymer; dioctylbutylamidotriazone (INCI: Diethylhexyl Butamidotriazone); 2,4-bis{[4-(2-ethylhexyloxy)-2-hydroxy]phenyl}-6-(4-methoxy-phenyl)-1,3,5-triazine; 2,4-bis[5-1(dimethylpropyl)-benzoxazol-2-yl-(4-phenyl)imino]-6-(2-ethylhexyl)-imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; piperazine derivatives; zinc oxide.

9. Cosmetic preparation according to any of the preceding claims, **characterized in that** the preparation comprises 2,4,6-tris[anilino-(p-carbo-2'-ethyl-1'-hexyloxy)]-1,3,5-triazine (INCI: Ethylhexyl Triazone) and/or 2,4-bis{[4-(2-ethylhexyloxy)-2-hydroxy]phenyl}-6-(4-methoxy-phenyl)-1,3,5-triazine (INCI: Bis-Ethylhexyloxyphenol methoxyphenyl Triazine).

10. Cosmetic preparation according to any of the preceding claims, **characterized in that** the preparation comprises one or more oils selected from the group of compounds Butylene Glycol Dicaprylate/Dicaprate, Phenethyl Benzoate, C12-15 Alkyl Benzoate, dibutyl adipate; diisopropyl adipate; diisopropyl sebacate, dicaprylyl carbonate, di-C12-13 Alkyl Tartrate, Butyloctyl Salicylate, Diethylhexyl Syringylidene Malonate, Hydrogenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate.

11. Cosmetic preparation according to any of the preceding claims, **characterized in that** the preparation comprises one or more compounds selected from the group of compounds glycyrrhetic acid, urea, arctiin, alpha-lipoic acid, folic acid, phytoene, D-biotin, coenzyme Q10, hyaluronic acid, alpha-glucosylrutin, carnitine, carnosine, caffeine, natural and/or synthetic isoflavonoids, glycerylglucose, creatine, creatinine, taurine, β-alanine and/or licochalcone A, panthenol, tocopherol, tocopherol acetate, vitamin C, vitamin C derivative, Glycyrrhiza inflata root extract, polidocanol, magnolol, honokiol, tocopheryl acetate, dihydroxyacetone; 8-hexadecene-1,16-dicarboxylic acid, glycerylglycose, (2-hydroxyethyl)urea, vitamin E or derivatives thereof, hyaluronic acid and/or salts thereof, licochalcone A.

## Revendications

1. Préparation cosmétique, contenant :
a) du salicylate d'octyle (Ethylhexyl Salicylat),
b) du dioxyde de titane selon la structure cristalline rutile ayant une taille de particule primaire de 2 à 100 nm, la préparation contenant exclusivement du dioxyde de titane selon la structure cristalline rutile, et le dioxyde de titane étant revêtu avec de la silice, le dioxyde de titane revêtu avec de la silice comprenant une couche de diméthicone sur le côté extérieur de la couche de silice, **caractérisée en ce que** la préparation est exempte de propylparabène, de butylparabène, de méthylisothiazolinone, de chlorométhylisothiazolinone, d'IPBC (IUPAC : carbamate de 3-iodo-2-propinylbutyle), de DMDM-hydantoïne, de diméthylol glycol, de diméthylol urée, de glycinate d'hydroxyméthyle de sodium, de BHT (IUPAC : 2,6-di-*tert-*butyl-4-méthylphénol), de 3-(4-méthylbenzylidène)camphre et de 2-hydroxy-4-méthoxybenzophénone.

2. Préparation cosmétique selon la revendication 1, **caractérisée en ce que** la préparation contient du salicylate d'octyle (Ethylhexyl Salicylat) en une concentration de 0,5 à 5 % en poids, par rapport au poids total de la préparation.

3. Préparation cosmétique selon l'une quelconque des revendications précédentes, **caractérisée en ce que** la taille de particule secondaire du dioxyde de titane selon la structure cristalline rutile ayant une taille de particule primaire de 2 à 100 nm est comprise entre 0,05 et 50 µm.

4. Préparation cosmétique selon l'une quelconque des revendications précédentes, **caractérisée en ce que** la préparation contient le dioxyde de titane selon la structure cristalline rutile ayant une taille de particule primaire de 2 à 100 nm en une concentration de 0,5 à 10 % en poids, par rapport au poids total de la préparation.

5. Préparation cosmétique selon l'une quelconque des revendications précédentes, **caractérisée en ce que** la préparation se présente sous la forme d'une émulsion.

6. Préparation cosmétique selon l'une quelconque des revendications précédentes, **caractérisée en ce que** la préparation contient un ou plusieurs émulsifiants choisis dans le groupe de composés constitué par le stéarate-citrate de glycéryle, le stéarate de glycéryle (auto-émulsifiant), l'acide stéarique, les sels de stéarate, le distéarate de polyglycéryl-3-méthylglycose, le sulfate de cétéaryle sodique, le glutamate de stéaroyle sodique, le stéarate de polyglycéryle-10, l'alcool cétéarylique, le sulfosuccinate de cétéaryle, le phosphate de cétyle potassique.

7. Préparation cosmétique selon l'une quelconque des revendications précédentes, **caractérisée en ce que** la préparation contient un ou plusieurs alcools choisis dans le groupe de composés constitué par l'éthanol, le 1,2-pentanediol, le 1,2-hexanediol, le 1,2-octanediol, le 1,2-décanediol, le 2-méthyl-1,3-propanediol, le phénoxyéthanol et/ou l'éthylhexylglycérine.

8. Préparation cosmétique selon l'une quelconque des revendications précédentes, **caractérisée en ce que** la préparation contient un ou plusieurs filtres UV, choisis dans le groupe de composés constitué par l'acide 2-phénylbenzimidazole-5-sulfonique et/ou ses sels, les sels de l'acide phénylène-1,4-bis-(2-benzimidazyl)-3,3'-5,5'-tétrasulfonique, le 1,4-di(2-oxo-10-sulfo-3-bornylidène-méthyl)-benzène et ses sels, les sels de l'acide 4-(2-oxo-3-bornylidène-méthyl)benzène-sulfonique, les sels de l'acide 2-méthyl-5-(2-oxo-3-bornylidène-méthyl)sulfonique, le 2,2'-méthylène-bis-(6-(2H-benzotriazol-2-yl)-4-(1,1,3,3-tétraméthylbutyl)-phénol), le 2-(2H-benzotriazol-2-yl)-4-méthyl-6-[2-méthyl-3-[1,3,3,3-tétraméthyl-1-[(tri-méthylsilyl)oxy]disiloxanyl]propyl]-phénol, le 3-(4-méthylbenzylidène)camphre, le 3-benzylidène-camphre, le 4-(tert.-butyl)-4'-méthoxydibenzoylméthane, l'acide téréphtalidène-dicamphre-sulfonique, l'acrylate de 2-éthylhexyl-2-cyano-3,3-diphényle, l'ester 2-éthylhexylique de l'acide 4-(diméthylamino)benzoïque, l'ester amylique de l'acide 4-(diméthylamino)benzoïque, l'ester di(2-éthylhexylique) de l'acide 4-méthoxybenzalmalonique, l'ester isoamylique de l'acide 4-méthoxycinnamique, la 2-hydroxy-4-méthoxybenzophénone, la 2-hydroxy-4-méthoxy-4'-méthylbenzophénone, la 2,2'-dihydroxy-4-méthoxybenzophénone, l'ester hexylique de l'acide 2-(4'-diéthylamino-2'-hydroxybenzoyl)-benzoïque, le salicylate d'homomenthyle, le 2-hydroxybenzoate de 2-éthylhexyle, le benzalmalonate de diméthicodiéthyle, le copolymère de 3-(4-(2,2-bis-éthoxycarbonylvinyl)-phénoxy)propényl)-méthoxysiloxane/diméthylsiloxane, la dioctylbutylamidotriazone (INCI : Diethylhexyl-Butamidotriazone), la 2,4-bis-{[4-(2-éthyl-hexyloxy)-2-hydroxy]-phényl}-6-(4-méthoxyphényl)-1,3,5-triazine, la 2,4-bis-[5-1-(diméthylpropyl)benzoxazol-2-yl-(4-phényl)-imino]-6-(2-éthylhexyl)-imino-1,3,5-triazine de n° CAS 288254-16-0), l'ester tris-(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone), la 2,4,6-tribiphényl-4-yl-1,3,5-triazine, la mérocyanine, les dérivés de pipérazine, l'oxyde de zinc.

9. Préparation cosmétique selon l'une quelconque des revendications précédentes, **caractérisée en ce que** la préparation contient de la 2,4,6-tris-[anilino-(p-carbo-2'-éthyl-1'-hexyl-oxy)]-1,3,5-triazine (INCI : Ethylhexyl Triazone) et/ou de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

10. Préparation cosmétique selon l'une quelconque des revendications précédentes, **caractérisée en ce que** la préparation contient une ou plusieurs huiles choisies dans le groupe de composés constitué par le dicaprylate/dicaprate de butylène glycol, le benzoate de phénéthyle, le benzoate d'alkyle en C12-15, l'adipate de dibutyle, l'adipate de diisopropyle, le sébacate de diisopropyle, le carbonate de dicaprylyle, le tartrate de di-alkyle en C12-13, le salicylate de butyloctyle, le malonate de diéthylhexyl-syringylidène, le dimérate d'huile de ricin hydrogénée, la triheptanoïne, le lactate d'alkyle en C12-13, le benzoate d'alkyle en C16-17, le caprylate de propylheptyle, le triglycéride caprylique/caprique, le 2,6-naphtalate de diéthylhexyle, l'octyldodécanol, le triglycéride caprylique/caprique, le cocoate d'éthylhexyle.

11. Préparation cosmétique selon l'une quelconque des revendications précédentes, **caractérisée en ce que** la préparation contient un ou plusieurs composés choisis dans le groupe de composés constitué par l'acide glycyrrhétinique, l'urée, l'arctiine, l'acide alpha-liponique, l'acide folique, le phytoène, la D-biotine, la coenzyme Q10, l'acide hyaluronique, l'alpha-glucosylrutine, la carnitine, la carnosine, la caféine, les isoflavonoïdes naturels et/ou synthétiques, le glycérylglucose, la créatine, la créatinine, la taurine, la β-alanine et/ou la licochalcone A, le panthénol, le tocophérol, l'acétate de tocophérol, la vitamine C, les dérivés de vitamine C, l'extrait de racine de Glycyrrhiza inflata, le polydocanol, le magnolol, l'honokiol, l'acétate de tocophéryle, la dihydroxyacétone, l'acide 8-hexadécène-1,16-dicarboxylique, le glycérylglycose, la (2-hydroxyéthyl)urée, la vitamine E et ses dérivés, l'acide hyaluronique et/ou ses sels, la licochalcone A.
